# DEMANDE DE BREVET EUROPEEN

(11) **EP 0 862 913 A1**
(43) Date de publication de la demande: **09.09.1998**
(21) Numéro de dépôt: 98400515.7
(22) Date de dépôt: 04.03.1998
(51) Int. Cl.: A61K 7/48, A61K 7/06

(54) **Composition huileuse homogène à base de gomme de silicone**

(30) Priorité: 04.03.1997 FR 9702538
(71) Demandeur: L'OREAL, 75008 Paris (FR)
(72) Inventeur: Bara, Isabelle, 75013 Paris (FR)
(74) Mandataire: Stalla-Bourdillon, Bernard

(57) **Abrégé**

Composition huileuse homogène destinée à un usage cosmétique.

Cette composition contient en mélange :
(a) au moins une gomme de silicone,
(b) au moins une huile végétale ou un triglycéride de synthèse, et
(c) au moins une silicone volatile de type alkylpolyméthylpolysiloxane correspondant à la formule suivante : 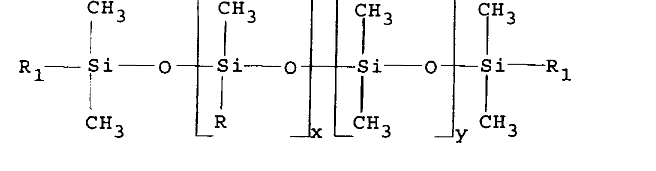
dans laquelle :
x = 0 à 10
y = 0 à 10
R représente un radical alkyle linéaire ou ramifié ayant de 5 à 10 atomes de carbone,
R₁ représente -CH₃ ou R, sous réserve que lorsque x = 0, R₁ représente R.

Utilisation de la composition huileuse homogène pour la réalisation de compositions cosmétiques sous forme anhydre ou sous forme dispersée en milieux aqueux.

## Description

L'utilisation de gommes de silicone en cosmétique présente un très grand intérêt dans la mesure où elles permettent de conférer plus de douceur, de glissant, de confort et de facilité d'étalement aux compositions les contenant.

Toutefois, l'utilisation de gommes de silicone présente l'inconvénient d'incompatibilité lorsque celles-ci sont associées à des huiles végétales ou à des triglycérides de synthèse dans la mesure où ces gommes forment presque immédiatement un précipité ou une séparation de phase.

On désigne sous le terme "gommes de silicone" des polydiméthyl siloxanes linéaires qui peuvent être hydroxylés ou phénylés et qui ont la consistance d'une huile épaisse ou d'un solide transparent par opposition avec les alkyl, alcoxy diméthicones qui, lorsqu'ils sont solides, présentent un aspect opaque cireux, mais peuvent également avoir l'aspect d'une huile limpide lorsque leur point de fusion est inférieur à la température ambiante.

Les gommes de silicone telles que définies ci-dessus ont des masses moléculaires très élevées, supérieures à 500.000, notamment comprises entre 500.000 et 2.000.000.

Parmi les dérivés de silicone, il convient également de signaler les résines de silicone qui sont des produits de réticulation de siloxanes résultant d'une hydrolyse et d'une polycondensation de siloxanes de formule (R)₃SiOCH₃ et Si(OCH₃)₄, R étant un groupement alkyle en C₁-C₆.

Le problème de la compatibilité des gommes de silicone avec d'autres types de produits huileux que les huiles végétales ou les triglycérides de synthèse tels que par exemple les silicones fluides volatiles a pu être résolu selon la demande EP 521 647 par l'emploi d'esters gras C₁₆-C₂₂ de l'acide citrique. L'emploi d'un mono, di ou tricitrate en tant qu'ingrédient essentiel a en effet permis, selon cette demande, d'obtenir des compositions cosmétiquement satisfaisantes.

Le problème selon la présente invention était par contre de résoudre l'incompatibilité des gommes de silicone dans les huiles végétales ou les triglycérides de synthèse. Après diverses études sur de nombreuses substances susceptibles d'agir en tant que co-solvant, on a constaté de façon inattendue et surprenante qu'une classe particulière de silicones volatiles permettait l'incorporation de gommes de silicone dans des huiles végétales ou des triglycérides de synthèse. L'utilisation de telles silicones volatiles, et plus particulièrement d'alkylpolyméthylpolysiloxanes, permet en effet d'obtenir des compositions huileuses parfaitement homogènes et limpides présentant par ailleurs, du fait de la présence d'au moins une huile végétale ou d'un triglycéride de synthèse, d'excellentes propriétés cosmétiques notamment quant à la douceur et à la facilité d'étalement ainsi que quant à l'activité au niveau des soins de la peau.

La présente invention a donc pour objet à titre de produit industriel nouveau une composition huileuse homogène destinée à un usage cosmétique, celle-ci contenant en mélange :
(a) au moins une gomme de silicone,
(b) au moins une huile végétale ou un triglycéride de synthèse, et
(c) au moins une silicone volatile de type alkylpolyméthylpoly-siloxane correspondant à la formule suivante : 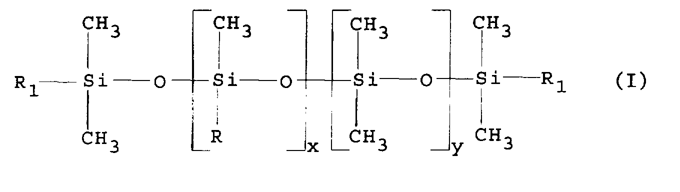
dans laquelle :
x = 0 à 10
y = 0 à 10
R représente un radical alkyle linéaire ou ramifié ayant de 5 à 10 atomes de carbone,
R₁ représente -CH₃ ou R, sous réserve que lorsque x = 0, R₁ représente R.

Les silicones volatiles telles que définies ci-dessus ont une masse moléculaire moyenne en poids généralement comprise entre 290 et 3.000, et une volatilité ou taux d'évaporation Nv généralement compris entre 100 et 800 secondes, et de préférence entre 150 et 500 secondes (temps correspondant à l'évaporation de 0,2 ml de silicone volatile à 23°C dans une atmosphère stable à 50 % d'humidité relative).

Parmi les silicones volatiles telles que définies ci-dessus pouvant être utilisées dans les compositions selon l'invention, on peut notamment citer le n-hexylheptaméthyltrisiloxane vendu par la Société DOW CORNING sous la dénomination de "DC1731®", le n-octylheptaméthyl-trisiloxane vendu sous la dénomination de "DC1732®" par la Société DOW CORNING.

La silicone volatile est généralement présente dans la composition huileuse selon l'invention en une quantité telle qu'elle permette d'obtenir une solution homogène et limpide de la gomme de silicone dans l'huile végétale ou les triglycérides de synthèse.

La silicone volatile est généralement présente en une proportion comprise entre 10 et 95 % en poids par rapport au poids total de la composition huileuse, mais de préférence entre 15 et 80 %.

La gomme de silicone des compositions huileuses selon l'invention peut avoir une masse moléculaire moyenne en poids comprise entre environ 500.000 et 2.000.000, et de préférence entre 800.000 et 1.500.000.

Parmi les gommes de silicone utilisables dans les compositions huileuses selon l'invention, on peut notamment mentionner celles correspondant à la formule suivante : 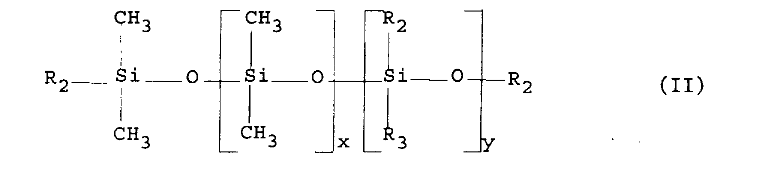 dans laquelle :
R₂ représente -CH₃, -OH ou -C₆H_{5,}
R₃ représente -CH₃, -OH, -C₆H₅ ou -OSi(CH₃)₃,
x = 0 ou un nombre entier et
y est un nombre entier sous réserve que y ou x et y soient des nombres entiers tels que la masse moléculaire moyenne en poids soit supérieure à 500.000, et de préférence comprise entre 500.000 et 2.000.000.

Parmi les gommes de silicone correspondant à la formule ci-dessus, on peut notamment citer la diphényldiméthicone, la phénylméthyl-diméthicone et la phényltriméthicone.

Ces gommes de silicone sont généralement commercialisées par les fournisseurs sous forme présolubilisée ou non dans une proportion de 5 à 20 %, et de préférence de 10 à 15 %, dans un polydiméthylsiloxane, linéaire ou cyclique, de faible poids moléculaire, volatil ou non volatil.

On peut citer à titre d'exemple en tant que gommes de silicone, la diphényldiméthicone à 15 % dans du cyclopentaméthylsiloxane vendue par la Société RHONE POULENC sous la dénomination de "Mirasil C-DPDM®", et les gommes de silicone vendues sous les dénominations de "SE30®" et "Viscasil®" par la Société GENERAL ELECTRIC, de "Q2-1403®" par la Société DOW CORNING et de "TP232®" par la Société OSI.

Dans les compositions huileuses selon l'invention, la gomme de silicone exprimée en matière sèche (MA) est généralement présente en une proportion de 0,03 à 30 % en poids, et de préférence entre 0,1 et 10 % en poids par rapport au poids total de la composition.

L'huile végétale selon l'invention est une huile à forte teneur en triglycérides ou essentiellement constituée de triglycérides d'esters d'acide(s) gras et de glycérol dont les acides gras peuvent être saturés ou insaturés, linéaires ou ramifiés, ayant de 6 à 40 atomes de carbone, mais de préférence de 8 à 30.

La teneur en triglycérides de l'huile végétale est d'au moins 80 % en poids et de préférence d'au moins 95 %.

Parmi les huiles végétales répondant à cette définition, on citera, selon l'invention, l'huile de germe de blé, l'huile de maïs, l'huile de karité, l'huile d'amandes douces, l'huile de ricin, l'huile de macadamia, l'huile d'abricot, l'huile de tournesol, l'huile de coton, l'huile de luzerne, l'huile de pavot, l'huile de potimarron, l'huile de sésame, l'huile de pépins de raisin, l'huile d'onagre, l'huile de millet, l'huile d'orge, l'huile de quinoa, l'huile de seigle, l'huile de carthame, l'huile de bancoulier, l'huile de passiflore, l'huile de rosier muscat et l'huile de pépins de cassis.

Les triglycérides de synthèse selon l'invention sont de préférence sous forme de mélanges et sont obtenus selon les méthodes connues de triestérification du glycérol à l'aide d'au moins un acide gras, saturé ou insaturé, linéaire ou ramifié, ayant de 6 à 40 atomes de carbone, mais de préférence de 8 à 30.

Parmi les triglycérides de synthèse, on peut notamment citer le trimyristate de glycéryle (trimyristin), le tripalmitate de glycéryle (tripalmitin), le trilinolénate de glycéryle (trilinolenin), le trilaurate de glycéryle (trilaurin), le tricaprate de glycéryle (tricaprin), le tricaprylate de glycéryle (tricaprylin) et leurs mélanges tels que notamment le tri(caprate/caprylate) de glycéryle vendu par la Société HÜLS AG sous les dénominations de "Miglyol 810®" et "Miglyol 812®" ou le tri(caprate/caprylate/linoléate) de glycéryle vendu par la Société HÜLS AG sous la dénomination de "Miglyol 818®".

Les huiles végétales et les triglycérides de synthèse selon l'invention se caractérisent par le fait qu'ils sont liquides à une température égale ou inférieure à 25°C.

La proportion en huile végétale ou en triglycérides de synthèse dans la composition huileuse selon l'invention est généralement comprise entre 0,5 et 89,97 % par rapport au poids total de la composition huileuse, mais de préférence entre 1 et 50 %.

Selon une variante de l'invention, la composition huileuse peut éventuellement contenir en outre en une proportion maximum de 50 % au moins une huile minérale ou une huile de synthèse choisie de préférence parmi les esters d'acides gras comme par exemple l'octanoate de stéaryle (huile de purcellin) , le myristate d'isopropyle, les octanoates, décanoates ou ricinoléates d'alcools ou de polyalcools, les alcools gras tels que par exemple l'octyl dodécanol ou l'alcool oléique, les huiles cosmétiques non polaires ou apolaires telles que les PDMS, les hydrocarbures, la vaseline, les polydécènes et le polyisobutène hydrogéné.

L'intérêt d'utiliser selon l'invention en tant que co-solvant une silicone volatile, telle que définie précédemment, présente l'avantage, lors de l'application de la composition, d'une élimination rapide, laissant alors subsister, sur la zone d'application, uniquement la gomme de silicone et l'huile végétale ou les triglycérides de synthèse.

La composition huileuse homogène selon l'invention peut être utilisée telle quelle sous forme anhydre ou encore sous forme dispersée en milieu aqueux lorsqu'elle constitue la phase grasse d'une émulsion du type eau-dans-l'huile ou huile-dans-l'eau.

Selon la première forme de réalisation, c'est-à-dire sous forme anhydre, les compositions cosmétiques selon l'invention peuvent alors se présenter sous forme d'une huile antisolaire (contenant un filtre solaire absorbant l'ultraviolet), d'une huile pour les mains, d'une huile pour le corps ou les cheveux, d'une huile de pré-rasage ou d'après-rasage, d'une huile pour le bain, dans divers types de conditionnements.

Selon cette première forme de réalisation de produits cosmétiques anhydres, la composition huileuse homogène selon l'invention peut constituer la phase grasse d'un produit de maquillage anhydre tel qu'un rouge à lèvres, un fond de teint compact, un blush ou un fard à paupières, la composition huileuse homogène étant présente en une proportion d'au moins 3 % en poids par rapport au poids total du produit cosmétique.

Selon la seconde forme de réalisation, c'est-à-dire sous forme dispersée, la composition huileuse homogène selon l'invention constitue la phase grasse d'une émulsion et représente environ 3 à 60 % en poids, la phase eau représentant de 20 à 96 %, et l'agent émulsionnant de 0,05 à 20 %, de préférence de 2 à 5 % en poids.

Les compositions cosmétiques selon l'invention peuvent également contenir divers ingrédients cosmétiques et notamment des agents conservateurs, des parfums, des antioxydants, des agents colorants, des filtres solaires, des pigments, des corps gras cireux, des agents gélifiants aqueux, ceux-ci étant présents en une proportion telle qu'ils ne nuisent pas à l'homogénéité de la composition.

Afin de mieux faire comprendre l'invention, on va maintenant donner à titre d'illustration plusieurs exemples de compositions huileuses homogènes et anhydres, ainsi que plusieurs exemples de compositions cosmétiques aqueuses contenant en tant que phase grasse la composition huileuse homogène selon l'invention.

### EXEMPLE 1 : Huile de soins du visage

On prépare selon l'invention une composition huileuse homogène pour les soins du visage sous forme limpide et incolore en procédant au mélange des ingrédients suivants :
- Huile de tournesol 13,65 %
- Huile de rosier muscat 0,87 %
- Huile de pépins de cassis 0,45 %
- Antioxydant 0,03 %
- N-hexylheptaméthyltrisiloxane vendu par la Société DOW CORNING sous la dénomination de "DC1731®" 20,00 %
- Gomme de silicone (diphényldiméthicone à 15 % dans du cyclopentaméthylsiloxane) vendue par la Société RHONE-POULENC sous la dénomination de "Mirasil C-DPDM®" 65,00 % (9,75 % MA)

### EXEMPLE 2 : Crème de soins sous forme d'une émulsion

On prépare selon l'invention une émulsion huile-dans-l'eau pour les soins de la peau en procédant au mélange des ingrédients suivants :

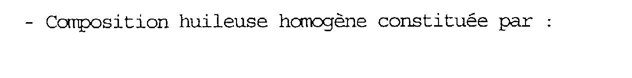

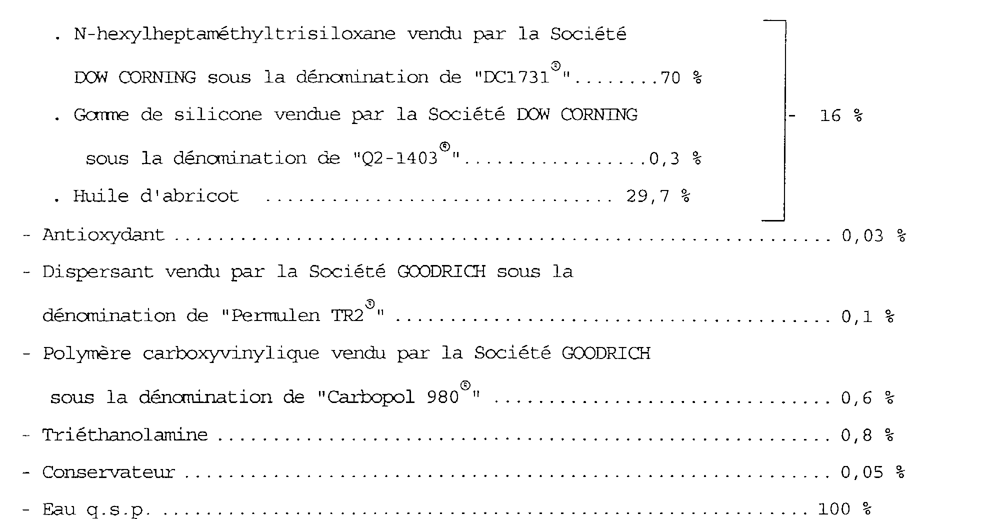

### Exemple 3 : Huile de soins du visage

On réalise une composition huileuse homogène selon l'invention en procédant au mélange des ingrédients suivants :
- Gomme de silicone (polydiméthylsiloxane hydroxylé) vendue par la Société DOW CORNING sous la dénomination de "SGM-36®" 1,00 %
- N-hexylheptaméthyltrisiloxane vendu par la Société DOW CORNING sous la dénomination de "DC 1731®" 69,00 %
- Huile d'abricot 10,00 %
- Tri (caprate/caprylate) de glycéryle vendu par la Société HÜLS AG sous la dénomination de "Miglyol 812®" 20,00 %

## Revendications

1. Composition huileuse homogène destinée à un usage cosmétique, caractérisée par le fait qu'elle contient en mélange :
(a) au moins une gomme de silicone,
(b) au moins une huile végétale ou un triglycéride de synthèse, et
(c) au moins une silicone volatile de type alkylpolyméthylpoly-siloxane correspondant à la formule suivante : 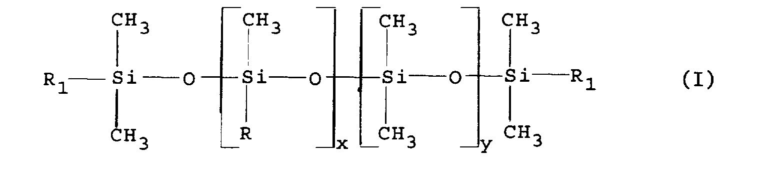
dans laquelle :
x = 0 à 10
y = 0 à 10
R représente un radical alkyle linéaire ou ramifié ayant de 5 à 10 atomes de carbone,
R₁ représente -CH₃ ou R, sous réserve que lorsque x = 0, R₁ représente R.

2. Composition selon la revendication 1, caractérisée par le fait que la silicone volatile du type alkylpolyméthylpolysiloxane a une masse moléculaire moyenne en poids comprise entre 290 et 3.000.

3. Composition selon la revendication 1 ou 2, caractérisée par le fait que la silicone volatile est choisie parmi le n-hexylheptaméthyl-trisiloxane et le n-octylheptaméthyltrisiloxane.

4. Composition selon l'une quelconque des revendications précédentes, caractérisée par le fait que la silicone volatile est présente en une proportion comprise entre 10 et 95 % en poids par rapport au poids total de la composition.

5. Composition selon la revendication 1, caractérisée par le fait que la gomme de silicone a une masse moléculaire moyenne en poids comprise entre environ 500.000 et 2.000.000.

6. Composition selon la revendication 1 ou 5, caractérisée par le fait que la gomme de silicone répond à la formule suivante : 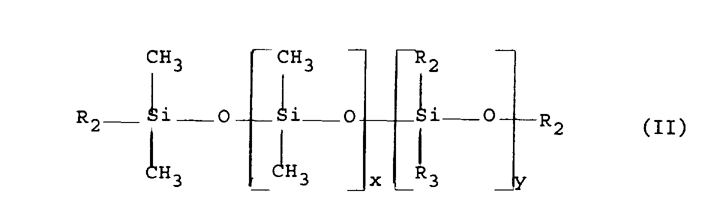 dans laquelle :
R₂ représente -CH₃, -OH ou -C₆H_{5,}
R₃ représente -CH₃, -OH, -C₆H₅ ou -OSi(CH₃)_{3,}
x = 0 ou un nombre entier et
y est un nombre entier sous réserve que y ou x et y soient des nombres entiers tels que la masse moléculaire moyenne en poids soit supérieure à 500.000.

7. Composition selon l'une quelconque des revendications 1, 5 et 6, caractérisée par le fait que la gomme de silicone est choisie parmi la diphényldiméthicone, la phénylméthyldiméthicone et la phényltriméthicone.

8. Composition selon l'une quelconque des revendications 1 et 5 à 7, caractérisée par le fait que la gomme de silicone exprimée en matière sèche est présente en une proportion de 0,03 à 30 % en poids par rapport au poids total de la composition.

9. Composition selon la revendication 1, caractérisée par le fait que l'huile végétale est une huile à forte teneur en triglycérides ayant au moins 80% en poids de triglycérides d'esters d'acide(s) gras et de glycérol, saturés ou insaturés, linéaires ou ramifiés ayant de 6 à 40 atomes de carbone.

10. Composition selon l'une quelconque des revendications 1 à 9, caractérisée par le fait que la proportion en huile végétale ou en triglycérides de synthèse est comprise entre 0,5 et 89,97 % par rapport au poids total de la composition.

11. Composition selon l'une quelconque des revendications précédentes caractérisée par le fait qu'elle est constituée de :
a) 0,03 à 30 % en poids d'au moins une gomme de silicone,
b) 0,5 à 89,97 % en poids d'au moins une huile végétale à forte teneur en triglycérides ou un triglycéride de synthèse, et
c) 10 à 95 % en poids d'au moins une silicone volatile de type alkylpolyméthylpolysiloxane de Formule (I) telle que définie à la revendication 1.

12. Composition cosmétique anhydre, caractérisée par le fait qu'elle est constituée par une composition huileuse homogène selon l'une quelconque des revendications 1 à 10, et contient éventuellement au moins un ingrédient cosmétique.

13. Composition cosmétique anhydre, caractérisée par le fait qu'elle contient une composition huileuse homogène selon l'une quelconque des revendications 1 à 11 en une proportion d'au moins 3 % en poids par rapport au poids total de la composition cosmétique.

14. Composition cosmétique sous forme dispersée notamment sous forme d'une émulsion huile-dans-l'eau ou eau-dans-l'huile, caractérisée par le fait que la phase grasse de ladite émulsion est constituée par une composition huileuse homogène selon l'une quelconque des revendications 1 à 11, et contient éventuellement au moins un ingrédient cosmétique.

15. Composition selon la revendication 14, caractérisée par le fait que la phase grasse représente de 3 à 60 % en poids, la phase eau de 20 à 96 % en poids, et l'agent émulsionnant de 0,05 à 20 % en poids par rapport au poids total de ladite composition.

16. Composition selon l'une quelconque des revendications 12 à 15, caractérisée par le fait qu'elle contient au moins un ingrédient cosmétique choisi parmi les agents conservateurs, les parfums, les antioxydants, les agents colorants, les filtres solaires, les pigments et les agents gélifiants.

17. Composition selon l'une quelconque des revendications 12 à 16 caractérisée par le fait qu'elle contient en outre au moins un corps gras cireux.
